# EUROPEAN PATENT APPLICATION

(11) **EP 2 990 060 A1**
(43) Date of publication of application: **02.03.2016**
(21) Application number: 15169865.1
(22) Date of filing: 29.05.2015
(51) Int. Cl.: A61L 2/18

(54) **CATHETER RINSING CHAMBER**

(30) Priority: 30.05.2014 US 201414291615
(71) Applicant: Morejon, Orlando, Key Largo, FL 33037 (US)
(72) Inventor: Morejon, Orlando, Key Largo, FL 33037 (US)
(74) Representative: Emde, Eric

(57) **Abstract**

The invention is directed towards a rinsing chamber for medical catheters. The device has at least an inlet port and an outlet port, arranged in such a fashion as to promote continuous flow of an irrigant fluid through the device and in contact with a predetermined portion of a catheter within the device, carrying away secretions, debris, or other contaminants from the exterior surface of the catheter.

## Description

### BACKGROUND OF THE INVENTION

### Field of the Invention

The invention is directed towards an apparatus for rinsing or decontaminating medical catheters and a method for using the same.

### DESCRIPTION OF THE RELATED ART

Many patients in a hospital and in particular, patients in an Intensive Care Unit ("ICU") must be fitted with tracheal tubes to facilitate their respiration. Specifically, a tracheal tube is an elongated, semi-rigid lumen which is inserted into a patient's nose or throat and projects into airflow communication with the patient's respiratory system. As such, the patient either directly, or with the aid of a respiratory unit, is able to breathe more effectively through the tracheal tube.

Recent studies have determined, however, that the accumulation of dried tracheo-bronchial secretions on the interior wall surface of an operating tracheal tube effectively decreases the lumen cross section, and thereby significantly increases the work of breathing for the intubated patient. Moreover, increasing the work of breathing for the patient necessitates that a higher level of support be provided to compensate and often results in the patient's incubation period and ICU stay being significantly prolonged. Furthermore, it is also seen that thick secretions on the walls of the tracheal tube often serve as a nidus for continued infection in the lungs, leading to added morbidity and hospital costs for the intubated patient.

Recently, new techniques of secretion removal have been developed that involve mechanical removal of secretions utilizing a flexible catheter with an inflatable scraping or scrubbing attachment meant to be inserted fully into an tracheal tube, inflated, and then pulled through the tracheal tube, bringing secretions with it. Reuse of these new cleaning catheters is desirable, as tracheal tube cleaning is a frequent and routine procedure among critical care staff. To that end, the present invention has been devised as an apparatus and method for rinsing or decontamination of primarily these, but also other medical catheters so that they may be reused.

### SUMMARY OF THE INVENTION

The present invention is directed towards a rinsing chamber with an irrigant inlet, an outlet port, and a catheter accommodation member. The rinsing chamber is structured and dimensioned to at least partially contain a predetermined portion of a medical catheter which is presented to the interior of the rinsing chamber and further stabilizes the catheter in a substantially axially orientation, or at least in an orientation that facilitates flow of the irrigant fluid in confronting relation with at least the external surface of the catheter to be rinsed. The catheter accommodation member may be at least an aperture through which the non-rinsed end of the catheter may protrude but can further include an elongated and at least partially hollow structure that further restricts the flow of fluid out of the aperture in the catheter accommodation member. The inlet port may be disposed on the distal end of the rinsing chamber, relative to the catheter accommodation member, and may be structured to accept the open end of a syringe and generally secure the syringe to the inlet port, such as by a Luer connection. The outlet port may be disposed closer to the proximal end of the rinsing chamber, but still distally of the catheter accommodation member, and is structured to accept generally a suction tube or other negative pressure vehicle. The interior surface of the rinsing chamber may also be lined with irregular structures, such as baffles, in order to promote turbulent flow of an irrigant around the catheter to be rinsed.

It is envisioned that the rinsing chamber may be manufactured as a two-part housing structured such that when both parts are engaged in an operative disposition, they form the rinsing chamber substantially as disclosed and that will be disclosed further. It will be appreciated to those skilled in the art that by having a two-part housing, a sealing structure correspondingly associated therewith is necessary to prevent irrigant fluid leaking from any compromise of the rinsing chamber structure caused by the two-part construction.

Use of the device is envisioned in a specific manner to maximize its efficiency in removing secretions and other contaminants. First the apparatus is disposed in an open configuration. Then a predetermined portion of a medical catheter such as, but not limited to, the distal end, is presented to the interior of the device. The device is then disposed in a closed configuration. Then an irrigation source and a negative pressure source are attached to the inlet port and outlet port, respectively. Next, the pressure in the rinsing chamber is lowered at least below atmospheric pressure. Then irrigant is introduced into the rinsing chamber, generally under pressure, and withdrawn concurrently to create a continuous flow of irrigant fluid through the rinsing chamber until the catheter is sufficiently irrigated. First drawing a vacuum and then introducing an irrigant fluid promotes preferential drainage through the outlet port and not the catheter accommodation member. Because it is not desirable to crimp the catheter, the catheter accommodation member may not form a tight seal around the catheter and if pressure is not reduced through the outlet port, contaminants may leak out of the catheter accommodation member. Furthermore, flushing irrigant into the device in a continuous manner promotes a unidirectional cross-flow of irrigant from distal to proximal end. This cross-flow is important to prevent contaminants breaking off one end of the medical catheter and reattaching at another end or creating a 'slurry' of debris and irrigant, but rather moves the irrigant from the cleaner, distal, end of the catheter towards the contaminated, proximal, end and out of the outlet port.

These and other objects, features and advantages of the present invention will become clearer when the drawings as well as the detailed description are taken into consideration.

### BRIEF DESCRIPTION OF THE DRAWINGS

For a fuller understanding of the nature of the present invention, reference should be had to the following detailed description taken in connection with the accompanying drawings in which:

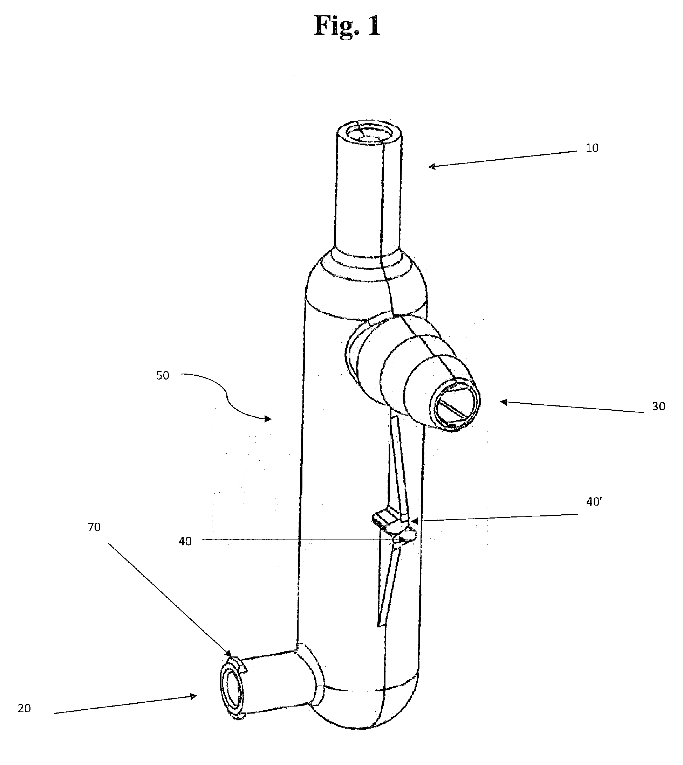

Figure 1 is a perspective view of one embodiment of the present invention in a closed disposition.
Figure 2 is a perspective view of one embodiment of the present invention in an open disposition.

Like reference numerals refer to like parts throughout the several views of the drawings.

### DETAILED DESCRIPTION OF THE PREFERRED EMBODIMENT

The preferred embodiment of the invention is a rinsing chamber 50 with an inlet port 20, outlet port 30, and catheter accommodation member 10 fixedly attached thereto. The catheter accommodation member 10 allows partial insertion of a medical catheter (not shown) such that only the desired end need be inside the device. The catheter accommodation member 10 includes at least an aperture 12 but in the primary embodiment also includes an elongated and at least partially hollow structure lined with ribs 11 on the interior thereof, the ribs 11 being dimensioned to form a loose seal around the catheter to avoid crimping or damaging the catheter. These ribs 11 also restrain the flow of irrigant fluid out of the catheter accommodation member 10.

The inlet port 20 may be disposed distally relative to the catheter accommodation member 10. The inlet port 20 includes at least a through hole penetrating into the rinsing chamber 50 but in the preferred embodiment also includes at least one irrigant source engagement member 70 to removably secure an irrigant source to the inlet port 20. It is envisioned that the irrigant source is generally a syringe filled with an irrigant, such as saline solution, and that the inlet port 20 and irrigant source engagement member 70 are dimensioned to receive the open end of a standard syringe. Accordingly, the irrigant source engagement member 70 may be of the Luer connector type in the interest of standardization. However, it is also envisioned in at least some other embodiments that the irrigant source is some other suitable vessel or container and may be much larger than a syringe in order to facilitate a prolonged continuous flow which assists in the expeditious rinsing of catheters by obviating the need to change or refill syringes multiple times.

Also in the preferred embodiment, the outlet port 30 is disposed on the proximal end of the rinsing chamber, relative to the inlet port 20, but distally of the catheter accommodation member 10. This relative positioning of the inlet port 20, outlet port 30, and catheter accommodation member 10, when combined with a negative pressure drawn on the outlet port 30, achieves the preferential evacuation of irrigant through the outlet port 30 and not through the catheter accommodation member 10. Accordingly, in at least one embodiment the outlet port 30 is structured and dimensioned to accept standard suction tubing which, for example, may be of a barb or 'push-on' type hose fitting.

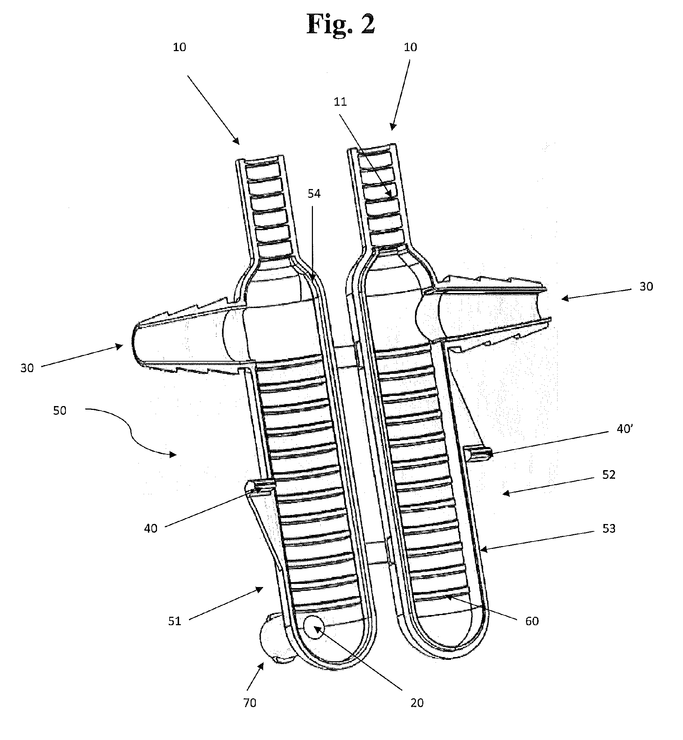

The preferred embodiment also has a rinsing chamber 50 at least partially made from a transparent material to facilitate visual inspection of the catheter during irrigation without removing the catheter or opening the rinsing chamber 50. It will be appreciated that the rinsing chamber 50 could also be made substantially of an opaque material but include a transparent viewing window or any other configuration that allows for visual confirmation of the catheter. Also in the preferred embodiment, the interior of the rinsing chamber 50 has an irregular surface in order to promote turbulent flow of the irrigant. As depicted in Fig. 2, the irregular surface is made of baffles 60 aligned along the minor dimension of the rinsing chamber 50, or at least partially perpendicular to the flow of irrigant fluid, but could be of a myriad combination of other shapes and/or orientations. The turbulent flow of the irrigant supplants the need to otherwise agitate the apparatus or catheter, as the chaotic character of turbulent flow is sufficient to dislodge secretions or other debris attached to at least the external surface of the catheter. Agitating the device is not desirable because agitation could cause contaminated irrigant to leak from the catheter accommodation member 10 due to the loose seal therein that avoids crimping the catheter.

With specific regard to Fig. 2, in the preferred embodiment as depicted therein, the rinsing chamber 50 is comprised of a first housing member 51 and a second housing member 52. The first housing member 51 and second housing member 52 have substantially identical footprints and are hingedly connected to facilitate disposition between an open configuration and a closed configuration. The open configuration facilitates presentation of the predetermined portion of the catheter to the interior of the rinsing chamber 50, while the closed disposition facilitates a thorough rinsing effect while containing and evacuating the irrigant fluid and any secretions or other debris attached to at least the external surface of the catheter.

Because the apparatus as depicted in Fig. 2 has both an open and closed configuration, it is desirable to include a means for releasably securing the apparatus in its closed position to ensure it does not inadvertently open during irrigation. Thus, at least one engagement member 40 is provided. As depicted, the engagement member 40 comprises a protrusion along the abutting plane of the first housing member, and a reciprocal engagement member 40' along the abutting plane of the second housing member. In the embodiment depicted in Fig. 2, the engagement member 40 is of a snap-fit type of closure, whereby the engagement member 40 is dimensioned and disposed to interfere and mechanically engage with a reciprocal engagement member 40' as the rinsing chamber 50 is disposed in closed configuration. It will be appreciated that many methods of releasably securing an apparatus that are known in the art are applicable to the apparatus herein disclosed. It is intended that the method depicted in Fig. 2 is illustrative and not limiting.

Also, with regard to Fig. 2, the outlet port 30 is molded or otherwise manufactured in two corresponding members, each correspondingly disposed on one of the first housing member 51 and second housing member 52 such that they form an enclosed channel when the rinsing chamber 50 is in a closed disposition. This configuration of the outlet port 30 allows the suction tubing to act as a secondary locking mechanism, should the engagement member 40 fail. The catheter accommodation member 10 is molded or otherwise manufactured in a similar fashion to the outlet port 30 -- two corresponding members, each correspondingly disposed on one of the first housing member 51 and second housing member 52 such that they form an enclosed channel when the rinsing chamber 50 is in a closed disposition - except that the purpose here is to facilitate presentation of the predetermined portion of the catheter to the interior of the rinsing chamber 50 while accommodating the externally remaining portion of the catheter

In at least one embodiment, the rinsing chamber includes structuring which provides an at least partially water-tight seal in the closed disposition. In the preferred embodiment, either of the first housing member 51 or second housing member 52 may have a raised lip 53 at least partially lining the interior perimeter of the first housing member 51 or second housing member 52. Correspondingly, the other of the first housing member 51 or second housing member 52 will have a corresponding lip recess 54, into which the raised lip 53 may fit when the rinsing chamber 50 is in a closed configuration. The raised lip 53 and correspondingly disposed lip recess 54 facilitate the abutting relation when manipulating the apparatus into a closed disposition by guiding the first housing member 51 and second housing member 52 into alignment, as well as contribute to an at least partially water-tight seal in the closed disposition.

In the preferred embodiment, the rinsing chamber 50 is dimensioned and sized in proportion to the catheter to facilitate movement of the fluid within the rinsing chamber 50 and generous contact of the fluid with at least exterior surface of the catheter. Generally this can be accomplished by dimensioning the rinsing chamber 50 on the order of ten times larger than the outer diameter of the catheter.

Because many modifications, variations, and changes in detail can be made to the described preferred embodiment of the invention, it is intended that all matters in the foregoing description and shown in the accompanying drawings be interpreted as illustrative and not in a limiting sense. Thus, the scope of the invention should be determined by the appended claims and their legal equivalents.

Now that the invention has been described,

## Claims

1. A device for irrigating medical catheters comprising:
an at least partially sealed rinsing chamber structured to at least partially contain a catheter to be rinsed;
an inlet port, structured to introduce an irrigant into said rinsing chamber;
an outlet port, structured to evacuate said irrigant from said rinsing chamber; and
said rinsing chamber, inlet port, and outlet port cooperatively structured and disposed to promote continuous flow of said irrigant through said inlet port, into said rinsing chamber, in confronting relation to the exterior surface portions of the catheter in a manner sufficient to facilitate removal of waste materials therefrom, and out of said outlet port.

2. The device of claim 1 wherein said rinsing chamber includes an interior surface, said interior surface comprises structuring which results in at least partially turbulent flow of the irrigant, through said rinsing chamber and in confronting relation to the external surface of the catheter to be rinsed.

3. The device of claim 2 wherein said structuring comprises baffles.

4. The device of claim 1 wherein said at least partially sealed housing is further structured to removably retain a catheter in a predetermined position within said rinsing chamber, said predetermined position facilitating exposure of at least the external surface of the catheter to the irrigant.

5. The device of claim 4 further comprising a catheter accommodation member comprising at least a channel structured and dimensioned to at least partially seal the catheter and restrict the flow of irrigant out of said catheter accommodation member.

6. The device of claim 1 wherein said inlet port comprises an irrigant source engagement member and through hole and is structured to accept and engage an irrigant source.

7. The device of claim 6 wherein said irrigant source engagement member comprises a Luer connecter for engagement of an irrigant source comprising a syringe.

8. The device of claim 1 wherein said outlet port comprises at least a channel which is structured and dimensioned to facilitate evacuation of the irrigant at least partially concurrent to input of the irrigant to establish a continuous flow of irrigant through said rinsing chamber.

9. The device of claim 8 wherein said outlet port is further structured for connection to a negative pressure source.

10. The device of claim 1 wherein said rinsing chamber is further structured and configured to stabilize said catheter in a predetermined orientation.

11. The device of claim 10 wherein said rinsing chamber includes an interior surface, said interior surface comprises structuring which supports at least partially a catheter in a predetermined orientation.

12. The device of claim 11 wherein said structuring comprises baffles.

13. The device of claim 1 wherein said rinsing chamber is at least partially composed of transparent material to facilitate visual inspection of said catheter.

14. The device of claim 1 wherein said rinsing chamber is dimensioned on the order of ten times larger the outer diameter of said catheter to be rinsed.

15. A device for irrigating medical catheters comprising:
a first and second housing member, hingedly connected for disposition between an open and closed configuration;
said closed configuration defined by said first and said second housing member disposed in abutting relation to form an at least partially enclosed rinsing chamber;
said open configuration defined by said first and said second housing member disengaged from said closed configuration in separated relation which facilitates presentation of at least a predetermined portion of a catheter to be rinsed to the interior of said rinsing chamber;
a catheter accommodation member comprising at least an aperture structured to at least partially accommodate the non-rinsed portion of a medical catheter;
an inlet port structured to facilitate introduction of an irrigant into said rinsing chamber; and
an outlet port structured to evacuate the irrigant and disposed in relation to said catheter accommodation member to preferentially evacuate the irrigant.

16. The device of claim 15 wherein said inlet port is disposed distally relative to said catheter accommodation member, and said outlet port is disposed distally relative to said catheter accommodation member and proximally relative to said inlet port.

17. The device of claim 15 wherein said outlet port is comprised of two channels, one of said two channels disposed on said first housing member, the other of said two channels disposed on said second housing member; each of said two channels disposed in relation to each other to form said outlet port when said first and said second housing members are disposed in said closed configuration.

18. The device of claim 15 wherein said catheter accommodation member is comprised of two channels, one of said two channels disposed on said first housing member, the other of said two channels disposed on said second housing member; each of said two channels disposed in relation to each other to form said catheter accommodation member when said first and said second housing members are disposed in said closed configuration.

19. The device of claim 18 wherein said catheter accommodation member further comprises an interior surface, said interior surface comprises a plurality of ribs structured and disposed to at least partially restrain the flow of the irrigant out of said catheter accommodation member.

20. The device of claim 15 wherein said first housing member further comprises a first engagement structure and said second housing member further comprises a second engagement structure; each of said first and said second engagement structures cooperatively structured and disposed to engage each other and releasably secure said first and said second housing member when disposed in said closed configuration.

21. The device of claim 15 wherein said rinsing chamber further comprises an interior surface, said interior surface comprises baffles.

22. A method of rinsing medical catheters comprising:
presenting at least a predetermined portion of a medical catheter to the interior of a rinsing chamber,
lowering the pressure in the interior of said rinsing chamber to below that of the immediate atmosphere,
introducing an irrigant under pressure to the interior of said rinsing chamber,
withdrawing the irrigant at least partially concurrent to the introduction thereof to establish a continuous flow of irrigant through said rinsing chamber, and
directing the continuous flow of irrigant through the rinsing chamber in confronting relation to at least the exterior surface portions of the catheter to facilitate removal of waste materials therefrom.
